# EUROPEAN PATENT APPLICATION

(11) **EP 3 539 619 A1**
(43) Date of publication of application: **18.09.2019**
(21) Application number: 18020099.0
(22) Date of filing: 15.03.2018
(51) Int. Cl.: A61Q 19/08, A61K 8/64, A61K 8/34, A61K 8/67, A61K 8/9789

(54) **CONCENTRATED COSMETIC COMPOSITION**

(71) Applicant: The Boots Company PLC, Nottingham NG90 1BS (GB)
(72) Inventor: HICKS, Jake Thomas, Chilwell, Nottingham NG9 6RR (GB); ELMS, Christopher John, Bottesford, Nottingham NG13 0FH (GB); BELL, Michael David, Higham, Alfreton, Derbys DE55 6EL (GB)
(74) Representative: Goodier, Claire-Louise

(57) **Abstract**

According to the present invention there is provided an aqueous concentrated cosmetic composition comprising at least 20% water, at least 4.5% by weight of the composition of humectant and high levels of skin benefit actives. The composition is used as a targeted area super concentrated serum or as a booster for addition to a singular dose of a second cosmetic composition.

## Description

### Technical Field

The present invention relates to concentrated cosmetic compositions and methods of using such cosmetic compositions. More particularly the present relates to concentrated cosmetic compositions for delivering high levels of moisturisation and anti-ageing benefits to the user.

### Background to the Invention

The Applicant has for many years been interested in research into the ageing process of skin and in developing skincare composition to combat the natural skin-related effects of ageing. In particular the Applicant has worked with renowned universities in the field of peptides and their effect on skin, and with peptide suppliers to manufacture peptides of interest. The Applicant has now developed a highly concentrated anti-ageing composition with both immediate and long term anti-ageing benefits.

As the skin ages, a number of changes can be seen. The skin begins to sag and lose elasticity owing to a loss of elastin and collagen. It loses plumpness owing to a loss of fat in the cheek, chin, nose and eye area and dehydration. The epidermis thins and the skin becomes more transparent, it loses the uniform tone and colour of youthful skin and hyperpigmentation and hypopigmentation spots appear. Hyperpigmentation spots are due to erratic melanocyte activity that can be the result of cumulative ultraviolet radiation. Hypopigmentation results from a loss of melanin and melanocytes in a given area.

The Applicant has been working on innovating a super concentrated anti-ageing serum or booster composition. However, it is not possible to simply increase the concentration of active ingredients in the composition. Raw materials, as bought from suppliers, are often less than 100% active and incorporate other ingredients such as solvent, emulsifiers, stabilising agent, preservatives and the like. This therefore means that if the skincare composition manufacturer were to increase the concentration of the active, they also increase the concentration of these other materials. This results in two problems; in the first the composition lacks 'space' for additional actives and in the second, high concentrations of some of these other ingredients can be irritant, skin drying or result in a poor aesthetic or feel of the composition.

Glycerin is often used as solvent or diluent in raw materials. High levels of glycerin are known to provide good moisturisation benefits. However glycerin is a highly viscous polyol and at high levels results in poor aesthetics, giving the composition a sticky, tacky feel. Tackiness is perpetually described as a problem in the cosmetic beauty industry and has been addressed in a number of ways.

US2013/0345316 relates to water-releasing emulsion-based cosmetic compositions. The application describes the issues associated with using high levels of humectant, such as glycerin, in that the composition has a sticky or tacky feel upon application to the skin and that this is undesirable to consumers. The application describes alternative solutions that may help the tacky feel such as light emollients and powders, but then concludes in reporting that these solutions do not solve the problem sufficiently to improve consumer appeal.

WO2001/00166 relates to skin care compositions and describes the problem of including high levels of skin benefit agents in compositions and the higher risk of associated negatives such as skin irritation stickiness and tackiness. The solution provided therein is a cosmetic composition comprising pantothenoic acid or derivatives thereof and a quaternary ammonium agent.

US2005/0058679 focuses on strengthening and maintaining the natural function of the skin as a barrier against environmental influences and endogenous substances. The application describes skin care products providing moisturisation by way of polyols, such as glycerol and sorbitol. This application also describes that polyol-containing products have poor sensory aesthetics, feeling tacky and greasy on the skin, and that a prior solution has been to incorporate expensive silicone oils and lipids. In this application the solution described is a cosmetic formulation comprising a polyol and a diol at specific percentage weights of the composition.

WO97/30679 describes a skin care composition comprising a quaternary ammonium compound, humectant and hydrophobic polymeric microspheres. The application describes the use of high levels of humectant in skin care moisturisation composition, but then goes on to explain that these leave the skin feeling greasy and/or tacky and are therefore undesirable. The problem is further exacerbated by increasing the relative amounts of these ingredients in the composition.

The Applicant has however discovered that tackiness in the product of the present invention and in its method of application is in fact a benefit and provides an advantage.

### Summary of Invention

According to the present invention there is provided a concentrated aqueous cosmetic composition comprising at least 20% water, comprising
a) at least 4.5% by weight of the composition of humectant
b) high levels of skin benefit active selected from the group consisting of peptides, tyrosinase inhibitor, vitamin B3, other vitamin, skin firming active, pigment and mixtures thereof wherein
   i. Where the active is a peptide the raw material active is present at at least 8% by weight of the composition;
   ii. Where the active is a tyrosinase inhibitor the raw material active is present at at least 3% by weight of the composition;
   iii. Where the active is a vitamin B3 or derivative the raw material active is present at at least 5% by weight of the composition;
   iv. Where the active is another vitamin selected from the group consisting of B1 to B12, with the exception of B3, vitamin E, vitamin D, vitamin K, vitamin H, vitamin A or mixtures thereof, the raw material active is present at at least 0.5% by weight of the composition;
   v. Where the active is a skin firming active, the raw material active is present at at least 5% by weight of the composition; and
   vi. Where the active is a pigment, the raw material active is present at at least 5% by weight of the composition.

### Detailed description of the Invention

The disclosed technology provides a composition, methods and uses as disclosed above.

### Aqueous Composition

The present invention is an aqueous composition. The composition preferably comprises from 20% to 80% by weight of the composition of water. In an alternative embodiment the composition comprises from 50% to 80% water. In another alternative embodiment the composition comprises from 63% to 79% water. The compositions may be free-flowing with viscosity of 1cps to 5000cps or alternatively the composition is thickened using a suitable rheology modifier. Preferably the composition is thickened. Preferably the composition has viscosity of from 5000cps to 50,000cps. In another embodiment, the composition is thickened and has viscosity of from 15,000cps to 30,000cps. Viscosity according to the present invention is measured according to the below described method.

### Humectant

The compositions of the present invention include a humectant at levels of at least 4.5%. In another embodiment the composition comprises at least 7% humectant. In another embodiment, the composition comprises at least 15% humectant by weight of the composition. In one embodiment the humectant is present at levels of at least 22% by weight of the composition.

By the term humectant we mean a substance that holds, retains and attracts water to or in the surface of the skin. The term is used with its normal cosmetic meaning.
Suitable examples of humectants include those selected from the group consisting of glycols such as glycerol, butylene glycol, propylene glycol, pentylene glycol, sorbitol, glucitol, mannitol, hydroxypropyl sorbitol, erythritol, threitol, pentaerythritol, xylitol, anhydroxylitol, xylitylglucoside, 1,2,6 hexanetriol, ethoxylated glycerin, propoxylated glycerin and mixtures thereof.

In one another embodiment the humectant is a polyol selected from the group consisting of glycerol, butylene glycol, propylene glycol, pentylene glycol, xylitol, anhydroxylitol, xylitylglucoside and mixtures thereof.

The high levels of humectant in the present composition provide a key advantage of the present composition over the prior art in that they deliver tackiness. Tackiness in prior art compositions is described as a problem, and a characteristic of a composition to be avoided and/or managed. The present composition is specifically designed as a product to treat a targeted area. The product is applied in small quantities to a specific problem area on the users face. The tackiness provides an advantage in that the product exhibits a high contact angle on the surface of the skin. The higher contact angle results in less spreading of the product, and therefore less migration from the targeted area. This is a clear advantage in that the concentrated product will act for longer in the area in which the consumers expects to see the benefit, for example a wrinkle, versus a product with a lower contact angle. This benefit is evidenced by the data presented herein in which the Applicant compared a product of the present invention with a product of similar composition and viscosity, but with lower contact angle and tackiness.

The present composition is also designed to be used as a booster, to be added to another second cosmetic product to boost functionality, for example to a day cream. In this embodiment the present product is diluted and the tackiness is lost. This however is not an issue, as the second product is then used as per normal usage instructions in which tackiness is not desirable. Hence tackiness is intentionally maintained when it is an advantage and lost when it is not desirable.

### Skin Benefit Actives

The compositions of the present invention comprise skin benefit actives at high levels. By the term high levels we mean levels which exceed those that the raw material manufacturer has tested for efficacy or has been used before topically. Skin benefit actives are selected from the group consisting of peptides, tyrosinase inhibitor, vitamin B3, another vitamin including vitamin B other than vitamin B3, E, A, K, H, D, skin firming agent, pigment and mixtures thereof.

Percentage as used below are percentage of the raw material in the composition, and not percentage of the active ingredient in the raw material, in the composition.

Where the active is a peptide, the active is present at at least 8% by weight of the composition. Where the active is a tyrosinase inhibitor the active is present at at least 3% by weight of the composition. Where the active is a vitamin B3 or derivative thereof, the active is present at at least 5% by weight of the composition. Where the active is another vitamin active, the active is present at at least 0.5% by weight of the composition. Where the active is a colour pigment, the active is present at at least 5% by weight of the composition. Where the active is a firming active, the active is present at at least 5% by weight of the composition.

It is conceivable within the scope of the present invention that a peptide, tyrosinase inhibitor, vitamin B3, another vitamins including vitamin B other than vitamin B3, E, A, K, H, D, skin firming agent and/or pigment may be present at a level which is not considered high or is out with the range of the present invention. In this scenario the ingredient is not to be considered as the skin benefit active of the present invention, but is instead included as a secondary benefit ingredient. In this scenario at least one skin benefit active will be present at a level which is considered high or is within the range of the present invention.

### Anti-ageing Peptides

Peptides are defined as compounds comprising an uninterrupted sequence of amino acids. Typically the peptides are of natural origin. Any suitable, skin-active peptide with anti-ageing benefits may be used herein. Preferred anti-ageing peptides are selected from the group consisting of dipeptides, tripeptides, tetrapeptides, pentapeptides and mixtures thereof.

A dipeptide comprises an uninterrupted sequence of two amino acids. Amino acids, as employed herein, include and encompass all of the naturally occurring amino acids, either in D or L configuration. Amino acids are commonly indicated with reference to the conventional three letter code and the sequence is read from left to right. The composition of the present may comprise a dipeptide chosen from acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide17, palmitoyl dipeptide 5 diaminobutyroyl hydroxythreonine, palmitoyl dipeptide 5 diaminohydroxybutyrate, palmitoyl dipeptide 7 and mixtures thereof.

In one embodiment the composition of the disclosed technology may comprise a tripeptide, or mixtures thereof. The tripeptide may be naturally occurring or of synthetic origin. Suitable tripeptide compounds include tripeptide 1, 2, 3, 4, 5, 6, 7, 8, 9, 10,11, 12, 13, 14, 15, 16, 17, 18, 19, 20, 21, 22, 23, 24, 25, 26, 27, 28 ,29, 30, 31, 32, 33, 34, 35, 36 ,37, 38, 39, 40, 41, 42, 43, 44, 45, 46, derivatives thereof, and mixtures thereof. The tripeptide comprise one or more His-based tripeptides. Particularly preferred tripeptides comprise one or more His-based tripeptides. However another suitable tripeptide may be Arg-Lys-Arg. Particularly preferred tripeptides are based on the structure Gly-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., Gly-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides in accordance with the present invention, (and therefore also the more generic term tripeptides). Derivatives of GHK-tripeptides in accordance with the present invention include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

Particularly preferred embodiments of tripeptides in accordance with the present invention include N-Acyl-Gly-His-Lys and most preferably, N-Palmitoyl-Gly-His-Lys. Preferred commercially available tripeptide and tripeptide derivative compriseing compositions include Biopeptide-CL from SEDERMA, Maxilip(R) from SEDERMA, Biobustyl(R) from SEDERMA.

The compositions of the disclosed technology may further comprise a tetrapeptide. The tetrapeptide may be one or more rigin-based tetrapeptides, one or more ALAMCAT-tetrapeptides or mixtures thereof. The tetrapeptide may be naturally occurring or of synthetic origin. Suitable tetrapeptides for use in the present composition include those chosen from tetrapeptide 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 34, 35, derivatives thereof and mixtures thereof. Rigin-based tetrapeptides of the disclosed technology may be based on the structure Gly-Gln-Pro-Arg (Rigin) and include its analogs and derivatives thereof. Rigin is a typical tetrapeptide.

Rigin-based tetrapeptides in accordance with the present invention are based on the structure Gly-Gln-Pro-Arg (Rigin) and include its analogs and derivatives thereof. Rigin is a preferred tetrapeptide. Analogs of the tetrapeptide rigin useful in accordance with the present invention include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc, Xaa-Xbb-Xcc-Pro, Xaa-Xbb-Pro-Arg, wherein Xaa-Xbb-Pro-Xcc, Xaa-Xbb-Xcc-Arg, Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-based tetrapeptides in accordance with the present invention, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO3H, SH or S-S.

Preferred commercially available sources of tetrapeptides include RIGIN, EYELISS, Haloxyl, and MATRIXYL 3000, which comprise between 50 to 500 ppm of palmitoyl-Gly-Gln-Pro-Arg, and other ingredients, such as peptides, chalcones and an excipient, commercially available from SEDERMA, France and Tego Pep 417 available from Evonik. These may be used as components of the present compositions. The combination of tripeptides and tetrapeptides, is particularly preferred. The preferred ratio of tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of tetrapeptide used when considered in terms of their amounts in parts per million, again based on overall weight of the composition. In a particularly preferred embodiment, the composition of the present invention comprise a tetrapeptide of the sequence Gly-Gln-Pro-Arg, its analogs and derivatives in combination with one or more tripeptide of the sequences Gly-His-Lys, its analogs and derivatives. The compositions of the disclosed technology may further comprise a pentapeptide, derivatives of thereof, and mixtures thereof. As used herein, "pentapeptide" refers to both the naturally occurring pentapeptide and synthesized pentapeptide. Also useful herein are naturally occurring and commercially available compositions that comprise pentapeptides. Suitable pentapeptides are those chosen from pentapeptide 1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof and mixtures thereof. Pentapeptides

The compositions of the present invention may optionally comprise a pentapeptide, derivatives of pentapeptides, and mixtures thereof. As used herein, "pentapeptides" refers to both the naturally occurring pentapeptides and synthesized pentapeptides. Also useful herein are naturally occurring and commercially available compositions that comprise pentapeptides. Suitable pentapeptides are those selected from the group consisting of pentapeptide1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof and mixtures thereof. Suitable pentapeptides for use herein are the pentapeptide, lys-thr-thr-lys-ser, Arg-asp-lys-tyr-val (pentapeptide -1) and derivatives thereof. A preferred commercially available pentapeptide derivative-comprising composition is Matrixyl which comprises 100 ppm of palmitoyl-lys-thr-thr-lys-ser and is commercially available from Sederma, France.

Where peptide is present in the composition disclosed herein, the peptide raw material may be present at at least 8% by weight of the composition. In another embodiment the peptide raw material is present at from 15% or 20% to 75% by weight of the composition. In another embodiment the peptide raw material active is present at from 15% to 65% by weight of the composition.

### Tyrosinase Inhibitor Active

The composition disclosed herein optionally comprise a tyrosinase inhibitor. By the term tyrosinase inhibitor we mean an ingredient capable of inhibiting or reducing the oxidative action of the tryosinase enzyme. When cells have been damaged, for example by exposure to ultra violet light, the tyrosinase enzyme is activated. Tyrosinase enzyme plays a critical role in the synthesis of melanin. This activation will result in an overproduction of pigment, and what is termed hyperpigmentation. Hyperpigmentation comes in many forms such as freckles, post-inflammatory hyperpigmentation, dark spots, age spots and melisma, an uneven skin tone usually presented by a brown darkening of the skin.

In one embodiment the tyrosinase inhibitor may be selected from the group consisting of: emblica, mangostin, sophora, a flavonoid, hydroxyphenoxy propionic acid, Morus Nigra, Salix Nigra, Flamingia Macrophylla, Pelargonium Inquinans, Cercidiphylum japonicum, ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof. The O-substituted ascorbic acid or derivative thereof may be an O-alk(en)yl ascorbic acid or derivative thereof. As used herein "alk(en)yl" is intended to mean alkyl or alkenyl (typically alkyl). The alk(en)yl may be acyclic or cyclic, typically acyclic. The acyclic group may be linear or branched, typically linear. Typically the O-substituted ascorbic acid or derivative thereof is an O-alkyl ascorbic acid, or derivative thereof. A preferred ascorbyl derivative is 3-0-ethyl ascorbic acid.

In one preferred embodiment the tyrosinase inhibitor comprises a tyrosinase inhibitor selected from the group consisting of ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof, and mixtures thereof. In another preferred embodiment, the tyrosinase inhibitor comprises an O-substituted ascorbic acid, preferably 3-0-ethyl ascorbic acid.

In an alternative preferred embodiment, the composition comprises a tyrosinase inhibitor selected from the group consisting of ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof, and mixtures thereof and a further tyrosinase inhibitor selected from the group consisting of emblica, sophora, Morus alba leaf extract and mixtures thereof.

In a further alternative embodiment the tyrosinase inhibitor is selected from the group consisting of ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof, and mixtures thereof and a further tyrosinase inhibitor selected from the group consisting of emblica.

In a further alternative embodiment the tyrosinase inhibitor is selected from the group consisting of ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof, and mixtures thereof and a further tyrosinase inhibitor selected from the group consisting of sophora.

In a further alternative embodiment the tyrosinase inhibitor is selected from the group consisting of ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof, and mixtures thereof and a further tyrosinase inhibitor selected from the group consisting of Morus alba leaf extract.

Alternatively, the tyrosinase inhibitor may be a mixture of ingredients selected from the group consisting of emblica, Sophora, 3-0-Ethyl Ascorbic Acid, mulberry (Morus Alba Leaf Extract) and mixtures thereof.

The emblica may be emblica officinalis, typically comprising over 40% by weight (typically 50-80 wt % of active in the raw material) of Emblicanin A. Emblicanin B, Pedunculagin and Punigluconin, and not more than about 1% by weight of flavonoids. The emblica may be Phyllanthus emblica.

The Sophora may be an extract of a small tree, and shrub in the pea family Fabaceae. The Sophora may be a Sophora Angustifolia Root Extract, a Sophora flavescens root extract, a Sophora Alopecuroides Extract, or Sophora japonica extract. The Sophora Angustifolia Root Extract may be an extract of the roots of the Chinese Sophora, Sophora angustifolia, or Leguminosae. In one embodiment Sophora is derived from Sophora Angustifolia Root Extract.

Typically the emblica may be Phyllanthus emblica and Sophora is derived from Sophora Angustifolia Root Extract.

Where tyrosinase inhibitor is present in the composition disclosed herein it may be present at from 3% to 30%, or 3% to 20%, or 3% to 10 % by weight of the composition.

### Vitamin B3 Active

Vitamin B3 and niacin, is the common name for nicotinic acid. The physiologically active form of niacin is niacinamide. Niacin and niacinamide (nicotinamide or nicotinic acid amide) function in the body as a component of two coenzymes: nicotinamide adenine dinucleotide (NAD) and nicotinamide adenine dinucleotide phosphate (NADP).

Niacinamide can be used in skincare compositions to deliver a variety of benefits including boosting collagen production, skin lightening, boosting skin immunity, improving epidermal barrier performance and reducing blotchiness, redness, uneven skin tone and sensitivity.

The compositions used in the present invention may comprise a safe and effective amount of a natural or synthetic vitamin B3 compound.

As used herein, "vitamin B3 compound" means a compound having the formula: 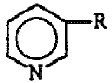 wherein R is - CONH2 (i.e., niacinamide), - COOH (i.e., nicotinic acid) or - CH2OH (i.e., nicotinyl alcohol); derivatives thereof; and salts of any of the foregoing.

Exemplary derivatives of the foregoing vitamin B3 compounds include nicotinic acid esters, including non-vasodilating esters of nicotinic acid, nicotinyl amino acids, nicotinyl alcohol esters of carboxylic acids, nicotinic acid N-oxide and niacinamide N-oxide.

Suitable esters of nicotinic acid include nicotinic acid esters of C1-C22, preferably C1-C16, more preferably C1-C6 alcohols. The alcohol may be straight-chain or branched chain, cyclic or acyclic, saturated or unsaturated (including aromatic), and substituted or unsubstituted. Preferred esters are those which do not yield a visible flushing response after application to the skin. Alternatively, a nicotinic acid material which does produce a flushing response can be used, if used at a lower dose to reduce the flushing effect. Non-flushing esters of nicotinic acid include tocopherol nicotinate and inositol hexanicotinate. Tocopherol nicotinate is preferred.

Other derivatives of the vitamin B3 compound are derivatives of niacinamide resulting from substitution of one or more of the amide group hydrogens. Non-limiting examples of derivatives of niacinamide useful herein include nicotinyl amino acids, derived, for example, from the reaction of an activated nicotinic acid compound (e.g., nicotinic acid azide or nicotinyl chloride) with an amino acid, and nicotinyl alcohol esters of organic carboxylic acids (e.g., C1 - C18). Specific examples of such derivatives include nicotinuric acid and nicotinyl hydroxamic acid.

Exemplary nicotinyl alcohol esters include nicotinyl alcohol esters of carboxylic acids salicylic acid, acetic acid, glycolic acid, palmitic acid and the like. Other suitable vitamin B3 compounds are selected from the group consisting of 2- chloronicotinamide, 6-aminonicotinamide, 6-methylnicotinamide, n-methyl- nicotinamide, n,n-diethylnicotinamide, n-(hydroxymethyl)-nicotinamide, quinolinic acid imide, nicotinanilide, n-benzylnicotinamide, n-ethylnicotinamide, nifenazone, nicotinaldehyde, isonicotinic acid, methyl isonicotinic acid, thionicotinamide, nialamide, 1-(3-pyridylmethyl) urea, 2-mercaptonicotinic acid, nicomol, niaprazine and mixtures thereof.

Examples of the above vitamin B3 compounds are well known in the art and are commercially available from a number of sources, e.g., the Sigma Chemical Company (St. Louis, MO); ICN Biomedicals, Inc. (Irvin, CA) and Aldrich Chemical Company (Milwaukee, WI).

The compositions of the present invention preferably comprise one or more vitamin B3 compounds. Preferred vitamin B3 compounds are niacinamide and tocopherol nicotinate. Niacinamide is more preferred.

Salts of the vitamin B3 compound are also useful herein. Non-limiting examples of salts of the vitamin B3 compound useful herein include organic or inorganic salts, such as inorganic salts with anionic inorganic species (e.g., chloride, bromide, iodide, carbonate, preferably chloride), and organic carboxylic acid salts (including mono-, di- and tri- Cl-C18 carboxylic acid salts, e.g., acetate, salicylate, glycolate, lactate, malate, citrate, preferably monocarboxylic acid salts such as acetate).

The vitamin B3 compound may be included as the substantially pure material, or as an extract obtained by suitable physical and/or chemical isolation from natural sources. The vitamin B3 compound is preferably substantially pure, more preferably essentially pure.

Where Vitamin B3 or a derivative thereof is present, the composition preferably comprises from above 0.1% to about 10%, more preferably from about 1% to about 7%. In one embodiment the Vitamin B3 active is present from about 3% to about 7% of the composition.

### Other Vitamin Actives

The composition of the present invention may comprise other vitamins or derivative thereof, other than a vitamin B3 active. The composition may comprise vitamin A, vitamin B, vitamin B derivatives, vitamin B1 to vitamin B12 and theirs derivatives, other than vitamin B3 discussed earlier. Compositions of the present invention may comprise vitamin K, vitamin K derivatives, vitamin H, vitamin D, vitamin D derivatives, vitamin E, vitamin E derivatives, such as tocopherol and tocopheryl acetate, and pro-vitamins thereof, such as panthenol and mixtures thereof. Vitamin C and derivatives thereof are intentionally not included in this section, as they are deemed to be tyrosinase inhibitors within the context of the present invention. The vitamin compounds may be included as the substantially pure material, or as an extract obtained by suitable physical and/or chemical isolation from natural (e. g., plant) sources. The compositions of the present invention preferably comprise from above 0.1% to 5%, more preferably from 0.5% to 4% of another vitamin active raw material. In one embodiment the other vitamin active raw material if present, is present at from 0.5% to 3% by weight of the composition.

### Skin Firming Active

The present composition may comprise a skin firming active. The EEMCO (European Expert Group on Efficacy Measurement of Cosmetics and other Topical Products) defines firmness as skins ability to resists extension and exhibit good elastic recovery. By skin firming active we mean an ingredient that by stimulating collagen or other extracellular matrix component production in the skin, or by increasing volume in the skin through water binding, exert a firming benefit and/or improvement in the viscoelastic properties of the skin.

Skin firming actives include peptides other than those anti-ageing peptides discussed above for example pea protein, Hyaluronic acid, Olea Europaea (Olive) Fruit Oil, Centella Asiatica Extract, Alchemilla, Lady's Thistle, Horsetail, Porphyra Umbilicalis Extract (and) Laminaria Digitata Extract (and) Ascophyllum Nodosum Extract (and) Crithmum Maritimum Extract (and) Enteromorpha Compressa Extract (and) Fucus Vesiculosus Extract, Terminalia Catappa leaf extract, Sambucus Nigra flower extract, Aesculus Hippocastanum (Horse Chestnut) Bark Extract, Lactobacillus/Streptococcus Thermophilus/Soybean Extract Ferment, Hibiscus and mixtures thereof. The Hibiscus active may be *Hibiscus sabdariffa, Hibiscus rosa sinensis or* Hibiscus Abelmoschus (may also be known as Abelmoschus moschatus).

Where present, the skin firming active is present in the present composition at from above 5% to about 30 more preferably from 7% to 17%. In one embodiment the skin firming active raw material if present, is present at from 10% to 15% by weight of the composition.

### Pigment

The active may be a pigment. By pigment we mean a material, preferably a solid insoluble particulate material, with the ability to provide colour or hue to the composition. The resulting composition is then suitable for use as a colour cosmetic targeted action or booster product.

The pigments of the present invention may be organic or inorganic, and may be coated or uncoated. Preferably the pigments have a particle size ranging from 0.05 to 150 microns, more preferably from about 20-100 microns.

Preferred inorganic pigments include titantium dioxide, iron oxides, ultramarines, chromium oxide, chromium hydroxide, and mixtures thereof.

Organic pigments are preferably various aromatic types including azo, indigoid, triphenylmethane, anthroquinone, and xanthine dyes which are designated as D&C and FD&C blues, browns, greens, oranges, reds, yellows, etc. Preferred organic pigments generally consist of insoluble metallic salts of certified color additives, referred to as the Lakes.

Also included herein may be interference pigments absorbing light in the 380 to 750nm range in the visible range of light spectrum depending on the desired colour. The colored interference pigments are selected from, but not limited to, a group of interference pigments consisting of: colored interference pigments showing an absorption minimum of about 570-590 nm (violet color); colored interference pigments showing at least an absorption minimum of about 590-620 nm (blue color); colored interference pigments showing at least an absorption minimum of about 620-750 nm (green color); colored interference pigments showing at least an absorption minimum of about 380-450 nm (yellow color); colored interference pigments showing at least an absorption minimum of about 450-495 nm (orange color); colored interference pigments showing at least an absorption minimum of about 495-570 nm (red color) or mixture thereof.

Further examples of suitable pigments include, pearlescent pigments, such as mica and bismuth oxychloride.

The pigment may be presented as a coating on a substrate particulate material. Suitable particle substrates include natural mica, synthetic mica, graphite, talc, kaolin, alumina flake, bismuth oxychloride, tin oxide, silica flake, glass flake, ceramics, titanium dioxide, CaSO4, CaCO3, BaSO4, borosilicate and combinations thereof. Preferred substrates are selected from the group consisting of mica, silica, alumina flakes and mixtures thereof.

Where present the raw material pigment active is present at least 5% of the composition. In an alternative embodiment, the pigment is preferably present at 5 % to 50% by weight of the composition. In an alternative embodiment, the pigment is preferably present at from 5% to 30%.

### Adsorbing Powder

Adsorbing powders are an optional, although preferred element of the present concentrated composition. By the term adsorbing powder it is meant a particulate which holds a thin film of material on an external or internal surface thereof.

The use of the adsorbing powder allows for the tackiness of the composition to be largely maintained and not absorbed, whilst facilitating a more aesthetically acceptable product. The adsorbing powder also improves the viscosity and substantivity of the composition

Where present, the adsorbing powder is present at a level of from 0.5% to 10% by weight of the composition. In an alternative embodiment the adsorbing powder is present at from 2% to 6% by weight of the composition.

In one embodiment the porous powder preferably has mean particle size of 1 to 50µm, more preferably 1 to 40µm and most preferably 1 to 30µm. Mean particles size is measured using a Beckman Coulter laser diffraction particle size analyser, using the dry powder module.

Any suitable adsorbing powder may be used herein. In one embodiment the powder is selected from the group consisting of Aluminum Starch Octenylsuccinate, Boron Nitride, Lauroyl Lysine, Mica, Talc, Cellulose, Tin Oxide, Titanium Dioxide, Zinc Oxide, Polyamide and mixtures thereof

Preferred adsorbing powders are selected from the group consisting of corn-based starch, tapioca-based starch, silicone elastomer particles, silicone elastomer resin powder bonded with silica, cellulose powders and mixtures thereof.

Particularly preferred adsorbing powders are selected from the group consisting of aluminium starch octenylsuccinate e.g. Dry Flo PC and Dry Flo Pure available from Akzo Nobel, tapioca starch and polymethylsilsesquioxane e.g. Dry-Flo TS available from Akzo Nobel, dimethicone/vinyl dimethicone crosspolymer and silica and butylene glycol e.g. EP-9801 Hydro elastomer powder available from Dow Corning), polymethylsilsesquioxane/silica crosspolymer e.g. Granpowder QSC available from Grant Industries, cellulose powders e.g. Tego Feel Green available from Evonik and mixture thereof.

### Rheology Modifier

The compositions of the present invention comprise a rheology modifier in a preferred embodiment. The rheology modifier may be selected from a viscosity modifying agent, gelling agent or mixtures thereof. Suitable rheology modifiers include acrylic acid polymers and copolymers for example those available commercially under the trade name Carbopol® or Ultrez® (both Lubrizol), or a taurate copolymer such as acryloyl methyl taurate-vinylpyrrolidone copolymers, or hydroxyethylacrylate/sodium acryloyldimethyl taurate copolymers, or modified celluloses e. g. hydroxyethylcellulose available commercially under the trade name Natrosol® (Hercules) or hydroxypropylmethyl cellulose, amine oxides, block polymers of ethylene oxide and propylene oxide (for example, those available from BASF Wyandotte under the trade name "Pluronic"®), PVM, MA, or a decadiene crosspolymer (available under the trade name Stabilez® 60), ethoxylated fatty alcohols, salt (magnesium chloride, sodium chloride), Ammonium Acryloyl dimethyl taurate/Vinyl pyrolidone Copolymer for example those sold under the trade name Aristoflex®AVC (Clariant), phthalic acid amide, xanthan gum, starch, sodium polyacrylate, polyvinyl alcohols, fatty alcohols and alkyl galactmanans available under the trade name N-Hance® from Hercules.

In one particularly preferred embodiment the rheology modifier is a polyacrylate polymer, more particularly Polyacrylate Crosspolymer-6, even more particularly is that sold under the trade name SepiMax Zen® by Seppic.

Where the composition comprises a rheology modifier, it is present at a level of at least 0.05%. In one embodiment, the rheology modifier is present at from 0.05% to 3%, more preferably from 0.1% to 2% by weight of the composition. In one embodiment, where present the rheology modifier is present at from 0.5% to 1.5% of the composition.

### Other Ingredients

In addition to the ingredients discussed above, the compositions of the present invention may also include a variety other ingredients.

The composition may comprise a matrix metalloproteinase inhibitor. The term "matrix metalloproteinase inhibitor" relates to all molecule and/or plant or bacterial extracts having an inhibitory activity on at least one of the matrix metalloproteinases expressed, synthesized, or activated by or in the skin. The family of the matrix metalloproteinases is formed of several well-defined groups on the basis of their resemblance regarding structure and substrate specificity (Woessner J. F., Faseb Journal, vol. 5, 1991, 2145).

The MMPi may be present at a level up to 10%, or 0.01% to 10%, or 0.1% to 5% or 0.25% to 2.5%, or 0.5% to 1% by weight of the composition.

The compositions disclosed herein may optionally comprise a salicylic acid compound, its esters, its salts, or combinations thereof. In one embodiment of the composition disclosed herein comprises a salicylic acid compound at 0.0001% to 25%, or 0.001% to 15%, or 0.01% to 10%, or 0.1% to 5%, and or 0.01% to 2%, by weight of the composition, of salicylic acid. In one embodiment the salicylic acid compound is salicylic acid.

Preservatives may be added to the composition such as benzoic acid, sodium benzoate, sorbic acid, potassium sorbate, 2-bromo2-nitropropane-1,3-diol (bronopol, which is available commercially under the trade name Myacide ®, benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, isothiazolone and sodium propyl paraben and mixtures thereof, suitably in an amount of from 0.01% to 10% by weight of the composition.

Sequestering agents may be added to the composition, such as ethylenediamine tetraacetic acid and salts thereof, typically in an amount of from 0 or 0.005% to 0.5% by weight of the composition.

The composition may also comprise suitable, cosmetically acceptable diluents, carriers and/or propellants such as dimethyl ether. The composition may also include pearlising agents such as stearic monoethanolamide and/or mica, suitably in an amount of from 0 or 0.01% to 10% by weight of the composition.

Perfumes may be added suitably in an amount of from 0 or 0.01% to 2% by weight of the composition, as may water soluble dyes such as tartrazine, suitably in an amount of from a trace amount such as 0 or 1x10⁻⁵ % to 0.1 % by weight of the composition.

The composition may also include pH adjusting agents such as sodium hydroxide, amino methyl propanol, triethanolamine, suitably in an amount of from 0 or 0.01 % to 10% by weight of the composition. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH between 3 and 10, between 4 and 8, or between 4.5 and 6.5.

### Packaging

The composition of the present invention may be packaged in any suitable vessel. In one embodiment the composition is packaged in an airless package comprising an actuating means. The actuating means may be for example a push button, pump or squeeze activation mechanism. In a squeeze action mechanism, the container is made from a resilient plastic, and is designed to be squeezed to apply pressure on the composition contained within the container. In a button actuated mechanism, the container is preferably a hard plastic or glass bottle and the actuation of the button works a suitable mechanism that pushes or pulls down a panel which in turn applies pressure on the composition within. In a pump actuated package, the package is preferably a hard plastic or glass bottle and the pump actuation means comprises a dip tube and pump. Compression of the pump creates a vacuum which when released forces composition into and up through the tube and out through the pump nozzle.

In one embodiment the opening through which composition is delivered is a fine hole or nozzle permitting the dispensing of only small quantities of product.

In one embodiment the composition may be packaged in a dual compartment package, comprising the composition of the present invention and at least one other composition. The other composition may be a cosmetic beauty product, such as for example a day, night or eye cream, moisturiser, or a colour cosmetic such as a foundation, BB or CC cream, or a sunscreen product.

In one embodiment the composition may form part of a patch or mask product. In this embodiment, the composition is loaded onto a substrate which is then applied to the face or body of the user and left for a period of 1-20 minutes, before being removed. The substrate may be any suitable woven or nonwoven substrate of any suitable material. In one embodiment the substrate is made using synthetic fibres of polyester, nylon, rayon, cotton, cellulose or derivative thereof or mixtures thereof.

### Method of Use

The present composition is a concentrated cosmetic composition. It has been designed to be used in two different ways. In one embodiment, the composition is a direct application focused treatment. The composition is applied directly to the area of treatment, for example lines and wrinkles, dark pigmentation spots, areas where firmness is required. The product is highly concentrated and only a few drops of composition are required per application. In one embodiment, the user is directed to apply between 1 and 5 drops, preferably 1 and 3 drops of the composition to the treatment area. The size of the drop is dependent on the package used. Typically however, the Applicant has used a package dispensing a drop of between 0.01 to 0.1ml.

Alternatively, in another embodiment a few drops of the composition are added to another second composition by the user. The other second composition may be a cosmetic beauty product, such as for example a day, night or eye cream, moisturiser, serum, or a colour cosmetic such as a foundation, BB or CC cream, or a sunscreen product. In one embodiment, the user is directed to apply between 1 and 5 drops, preferably between 1 and 3 drops of the composition into a unitised dose of a second composition. A unitised dose is defined as being the quantity used by the user in a single application.
Alternatively, in one embodiment the composition may form part of a patch or mask product. In this embodiment, the composition is loaded onto a suitable substrate which is then applied to the face or body of the user and left for a period of 1-20 minutes, before being removed.

### Experimentation

The experiments of the present invention are not designed to be limiting, but are examples of the methodology used.

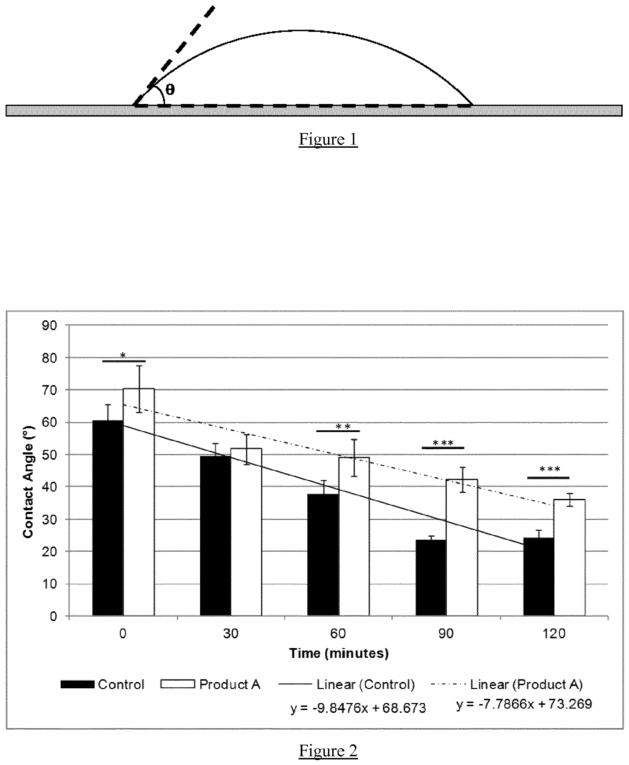

The compositions of the present invention are unusually tacky and exhibit a higher contact angle than similar compositions without the high level of humectant irrespective of composition viscosity. The composition (product A) has a higher contact angle for a longer period of time versus a composition of similar composition with similar viscosity, but without the same levels of tack (control), see figure 2. The compositions were prepared as follows:

| Raw Material | Product A | Control | Chemical | Product A | Control |
|---|---|---|---|---|---|
| Purified Water | q.s | | Aqua | q.s | q.s |
| Tetrasodium EDTA | 0.05 | 0.05 | Aqua | 0.007 | 0.007 |
| | | | Tetrasodium EDTA | 0.043 | 0.043 |
| Polyacrylate crosspolymer-6 | 0.6 | 0.6 | Polyacrylate crosspolymer-6 | 0.57 | 0.57 |
| | | | T-butyl alcohol | 0.012 | 0.012 |
| | | | Aqua | 0.018 | 0.018 |
| Aluminium starch octenylsuccinate | 3 | 3 | Aqua | 0.42 | 0.42 |
| | | | Aluminum starch octenylsuccinate | 2.58 | 2.58 |
| Potassium Hydroxide Solution | 0.04 | 0.04 | Aqua | 0.02 | 0.02 |
| | | | Potassium hydroxide | 0.02 | 0.02 |
| Phenoxetol Nipa | 0.6 | 0.6 | Phenoxyethanol | 0.6 | 0.6 |
| Capryly glycol and ethylhexylglycerin | 0.2 | 0.2 | Caprylyl glycol | 0.14 | 0.14 |
| | | | Ethylhexylglycerin | 0.06 | 0.06 |
| Acetyl Dipeptide-1 | 10 | 10 | Aqua | 2.5 | 2.5 |
| Cetyl Ester | | | Butylene glycol | 6.82 | 6.82 |
| | | | Sorbitan laurate | 0.5 | 0.5 |
| | | | Hydroxyethylcellulos e | 0.10 | 0.10 |
| | | | Acetyl dipeptide-1 cetyl ester | 0.08 | 0.08 |
| Purified Water | - | 19.42 | Aqua | - | 19.42 |
| Carbomer | - | 0.18 | Carbomer | - | 0.176 |
| | | | Aqua | - | 0.004 |
| 1,3-Butylene glycol | - | 3.6 | Aqua | - | 0.018 |
| | | | Butylene glycol | - | 3.582 |
| Polysorbate 20 | - | 0.09 | Polysorbate 20 | - | 0.09 |
| Sodium Hydroxide Solution | | 0.1232 | Aqua | - | 0.08624 |
| | | | Sodium hydroxide | - | 0.03696 |
| Lactic Acid Solution | - | 0.12726 | Aqua | - | 0.0152712 |
| | | | Lactic acid | - | 0.1119888 |
| Glycerin | 10 | - | Aqua | 0.02 | - |
| | | | Glycerin | 9.98 | - |
| Palmitoyl tripeptide/tetrapeptide 3 | 18 | - | Glycerin | 9.44 | - |
| | | | Aqua | 4.50 | - |
| | | | Polysorbate 20 | 0.09 | - |
| | | | Palmitoyl tripeptide-1 | 0.0054 | - |
| | | | Palmitoyl tetrapeptide-7 | 0.0027 | - |
| | | | Sodium lactate | 0.18 | - |
| | | | Carbomer | 0.18 | - |
| | | | Butylene glycol | 3.60 | - |

### Viscosity Measurement Procedure

Viscosity as described in the present is measured using a Brookfield LVDV-I Prime E viscometer plus Model G Laboratory Stand, equipped with LV Spindle 64. Viscosity measurements were obtained as follows:
1. Ensure the sample product has a temperature of 23°C and that it is not aerated. Sample is presented in a 60ml capacity plastic container with a diameter of 35mm and a height of 70mm.
2. Before measurement, auto-zero the viscometer after switching on the unit by following the on-screen instructions with no spindle attached to the viscometer
3. Select the spindle 64
4. Select the revolution speed "12". This will rotate the spindle at 12 revolutions per minute (rpm)
5. Carefully attach the spindle to the lower shaft of the viscometer
6. Lower the spindle into the sample product until the fluid level is at the immersion groove on the spindle shaft by turning the height adjustment knob on the right hand side of the viscometer mounting bracket. Ensure that the spindle is not in contact with the sides or base of the sample container
7. Press the "Timed Option" buttons
8. Use the Up and Down arrows to select the "Timed Stop" option then press "Enter" to confirm
9. Use the Up and Down arrows to select zero minutes, then press "Enter" to confirm
10. Use the Up and Down arrows to select 30 seconds , then press "Enter" to confirm
11. Press the "Motor On/Off' button to begin the measurement
12. The viscometer will display a countdown from 30 seconds, after which it will display the final viscosity measured
13. Record the viscosity reading

### Tackiness

Users were asked to use the product and were asked the extent to which they agree with the statement "This product did not leave my skin feeling sticky". The users were requested to choose from a 4 point scale, as follows:
1. Strongly disagree
2. Slightly disagree
3. Slightly agree
4. Strongly agree

Data for category 3 and 4 are combined as users who are in agreement with the statement. The tackiness score is the percentage of users in agreement with the statement. Therefore a high percentage score indicates a product which is not considered tacky, whereas a low score is a product which is considered tacky.

### Contact Angle Measurement

Contact angle for each composition was measured as follows. Using a GILSON MICROMAN M25 3-25UL positive displacement pipette, transfer 25µl of control/product A sample onto the centre of a glass microscope slide. We used a Menzel-Glaser, with dimensions 76 x 26 mm, available from Fisher Scientific).

Take an image of the droplet on the slide using a standard digital camera with the camera positioned at 0 degrees to the plane of the microscope slide along the longest edge of the slide.

Measure contact angle using the angle tool in the image analysis software imageJ (Applicant used v1.48) by drawing a horizontal line across the base of the droplet and an angled line tangential to the edge of the droplet at the point of contact to the slide and measuring the angle between (see figure 1). Measure both left and right contact angles and take the average of the two to give reported contact angle. The contact angle is the internal angle between the two lines. Figure 1 shows the positioning of lines to measure contact angle where: thick line is the microscope slide, thin line shows the droplet surface, dotted line shows the tangent to droplet surface at slide water interface and internal angle is identified.

The results of the contact angle testing are shown in figure 2. Product A exhibits a higher contact angle than the control sample at time zero minutes. The contact angle continues to be more pronounced over time. This means that the composition of the present invention is spreading less and therefore migrating less than the control sample. By consequence, because the product stays in place in the targeted area, it is more effective and the actives have longer to act on the target area.

### User Trial Data

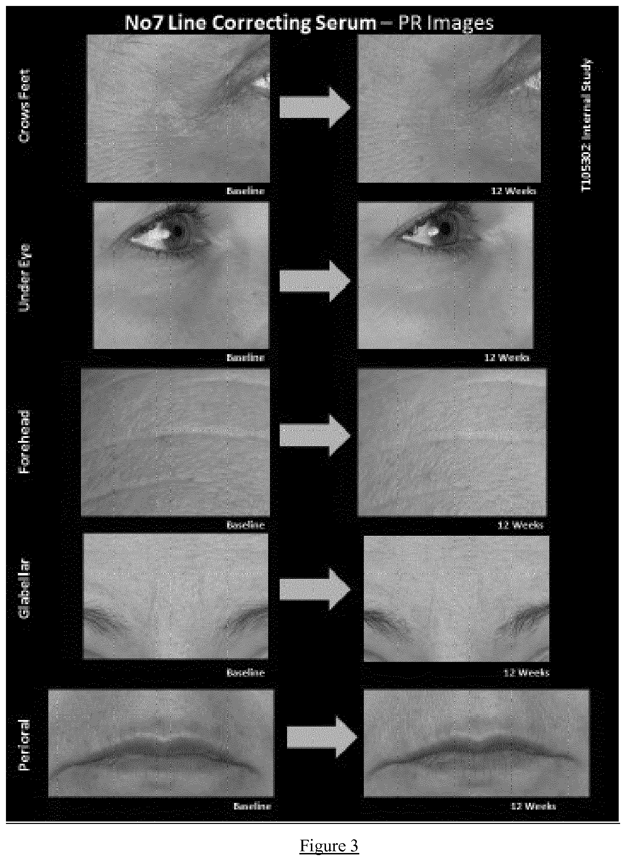

Consumers were asked to trial composition 1 below according to the invention over a period of 12 weeks. The consumers were selected by virtue of their keen interest in skincare and desire for younger looking skin. Results of the user trial are shown in the images of figure 3. The images are photographs of a selected participant before the trial began and after 12 week usage. The effects of usage are evident from the reduction in visibility of wrinkles and lines on the face of the participant.

| Raw Material | w/w% | Chemical | w/w% |
|---|---|---|---|
| Purified Water | q.s. | Aqua | q.s |
| Acetyl Dipeptide-1 Cetyl Ester | 10 | Aqua | 2.5 |
| | | Butylene glycol | 6.82 |
| | | Sorbitan laurate | 0.5 |
| | | Hydroxyethylcellulose | 0.1 |
| | | Acetyl dipeptide-1 cetyl ester | 0.08 |
| Tetrasodium EDTA | 0.05 | Aqua | 0.007 |
| | | Tetrasodium EDTA | 0.043 |
| Polyacrylate crosspolymer-6 | 0.6 | Polyacrylate crosspolymer-6 | 0.57 |
| | | T-butyl alcohol | 0.012 |
| | | Aqua | 0.018 |
| Aluminium starch octenylsuccinate | 3 | Aqua | 0.42 |
| | | Aluminum starch octenylsuccinate | 2.58 |
| Potassium hydroxide solution | 0.04 | Aqua | 0.02 |
| | | Potassium hydroxide | 0.02 |
| Phenoxyethanol | 0.6 | Phenoxyethanol | 0.6 |
| Capryly glycol and ethylhexylglycerin | 0.2 | Caprylyl glycol | 0.14 |
| | | Ethylhexylglycerin | 0.06 |
| Glycerin | 10 | Aqua | 0.02 |
| | | Glycerin | 9.98 |
| Palmitoyl tripeptide/tetrapeptide3 | 18 | Glycerin | 9.44 |
| | | Aqua | 4.50 |
| | | Polysorbate 20 | 0.09 |
| | | Palmitoyl tripeptide-1 | 0.0054 |
| | | Palmitoyl tetrapeptide-7 | 0.0027 |
| | | Sodium lactate | 0.18 |
| | | Carbomer | 0.18 |
| | | Butylene glycol | 3.60 |

In a second user trial participants either who have used botox in the past or were considering using botox as a solution to improving the appearance of wrinkles were asked to use composition 1.

The participants trialled the product for 8 success weeks and were asked questions at the time intervals indicated below. The users were requested to choose from a 4 point scale, as follows:
1. Strongly disagree
2. Slightly disagree
3. Slightly agree
4. Strongly agree

The score is as a percentage of their agreement to the statement.

| Statement | First use | 1 week | 2 weeks | 4 weeks | 8 weeks |
|---|---|---|---|---|---|
| | | | | | |
| It gave me better results than I thought possible with skincare | 63.55 | 69.85 | 72.35 | 73.4 | 78.25 |
| It gave me the results I thought only possible with Botox | 36.70 | 49.50 | 54.70 | 55.10 | 72.65 |
| Botox can wait | 54.85 | 62.95 | 55.30 | 70.40 | 74.90 |

The results of this second study show a surprising acceptance of a skin care product from a group of consumers that are very discerning and would normally have used considerably more invasive treatment to address their needs. This acceptance increases dramatically over the 8 week period, as the product has had time to work for longer. The success of the product results from the ability of the product to stay where it is needed in the targeted area and the high level of active ingredients.

## Claims

1. A concentrated aqueous cosmetic composition comprising at least 20% water, comprising
a) at least 4.5% by weight of the composition of humectant
b) high levels of skin benefit active selected from the group consisting of peptides, tyrosinase inhibitor, vitamin B3, other vitamin, skin firming active, pigment and mixtures thereof wherein
i) Where the active is a peptide the raw material active is present at at least 8% by weight of the composition;
ii) Where the active is a tyrosinase inhibitor the raw material active is present at at least 3%% by weight of the composition;
iii) Where the active is a vitamin B3 or derivative the raw material active is present at at least 5% by weight of the composition;
iv) Where the active is another vitamin selected from the group consisting of B1 to B12, with the exception of B3, vitamin E, vitamin D, vitamin K, vitamin H, vitamin A or mixtures thereof, the raw material active is present at at least 0.5% by weight of the composition;
v) Where the active is a skin firming active, the raw material active is present at at least 5% by weight of the composition;
vi) Where the active is a pigment, the raw material active is present at at least 5% by weight of the composition.

2. A composition according to the preceding claim wherein the humectant is selected from the group consisting of glycerol, butylene glycol, propylene glycol, pentylene glycol, xylitol, anhydroxylitol, xylitylglucoside, and mixtures thereof.

3. A composition according to any preceding claim wherein the humectant is glycerol.

4. A composition according to any preceding claim wherein the composition comprises peptides selected from dipeptides, tripeptides, tetrapeptides and mixtures thereof.

5. A composition according to any preceding claim wherein the composition comprises a tyrosinase inhibitor selected from the group consisting of ascorbyl glucoside, glyceryl ascorbate, myristyl 3-glyceryl ascorbate, aminopropyl ascorbyl phosphate, ascorbic acid, ascorbyl palmitate, magnesium ascorbyl phosphate, sodium ascorbyl phosphate, O-substituted ascorbic acid or derivative thereof, and mixtures thereof and a further tyrosinase inhibitor selected from the group consisting of emblica, sophora, Morus alba leaf extract and mixtures thereof.

6. A composition according to any preceding claim wherein the composition comprises vitamin B3 niacinamide.

7. A composition according to any preceding claim wherein the composition comprises another vitamin selected from the group consisting of tocopherol, tocopheryl acetate, panthenol and mixtures thereof.

8. A composition according to any preceding claim wherein the composition comprises a skin firming active selected from the group consisting of extracts of *Hibiscus sabdariffa, Hibiscus rosa sinensis,* Hibiscus Abelmoschus and mixtures thereof.

9. A composition according to any preceding claim wherein the composition comprises a pigment selected from 5% to 50% by weight of the composition.

10. A composition according to any preceding claim comprising an adsorbing powder.

11. A composition according to any preceding claim wherein the adsorbing powder is selected from the group consisting of aluminium starch octenyl succinate, boron nitride, lauroyl lysine, mica, talc, cellulose, tin oxide, titanium dioxide, zinc oxide, polyamide and mixtures thereof.

12. A composition according to any preceding claim wherein the adsorbing powder is present at a level of from 0.5% to 10% by weight of the composition.

13. A composition according to any preceding claim additionally comprising a rheology modifier selected from the group consisting of polyacrylate polymer and copolymer, taurate copolymers, modified cellulose, block polymers of ethylene oxide and/or propylene oxide, ethoxylated fatty alcohols, ammonium acryloyl dimethyl taurate/vinyl pyrolidone copolymer, xanthan gum, starch, polyvinyl alcohol and mixtures thereof.

14. A composition according to claim 13 wherein the rheology modifier is polyacrylate crosspolymer 6.

15. A method of treating a targeted area on the face of a user by applying to said targeted area a maximum of 5 drops of the composition according to any preceding claim.

16. A method of boosting the efficacy of a cosmetic product by adding to said product a maximum of 5 drops of the composition according to any of claims 1 to 6.
